# EUROPEAN PATENT APPLICATION

(11) **EP 3 571 990 A1**
(43) Date of publication of application: **27.11.2019**
(21) Application number: 19176086.7
(22) Date of filing: 23.05.2019
(51) Int. Cl.: A61B 5/06, A61B 5/00

(54) **POSITION SENSOR ON BRAIN-CLOT REMOVAL SHEATH AND LOCATION PAD COLLAR**

(30) Priority: 24.05.2018 US 201862675952 P; 15.01.2019 US 201916248393
(71) Applicant: Biosense Webster (Israel) Ltd., Yokneam, 2066717 (IL)
(72) Inventor: GOVARI, Assaf, 2066717 Yokneam (IL)
(74) Representative: Small, Gary James

(57) **Abstract**

A location tracking system includes a location pad collar and a processor. The location pad collar is configured to be placed around a neck of a patient and includes one or more magnetic field generators configured to generate one or more magnetic fields within a head of the patient. The processor is configured to receive, in response to the one or more generated magnetic fields, one or more position signals that are indicative of a location of a distal end of a sheath of a probe inside a blood vessel in a brain of the patient. Based on the received position signals, the processor is configured to estimate the location of the distal end of the sheath in the brain relative to a clot in the brain.

## Description

### FIELD OF THE INVENTION

The present invention relates generally to catheter-based position-sensing systems, and particularly to magnetic-field based position-sensing systems for minimally invasive intervention at a blood vessel in the brain.

### BACKGROUND OF THE INVENTION

Catheter designs that include a capability of indicating of a position of the catheter in a blood vessel in the brain have been proposed in the patent literature. For example, U.S. Patent Application Publication 2010/0113919 describes a catheter arrangement for insertion into a blood vessel. The catheter arrangement has a catheter with a proximal catheter tip, in which an intervention tool is guided to remove a blood clot from the blood vessel. The intervention tool has an element for trapping a blood clot, in particular a spiral, in the region of its tip. With a view to minimizing x-ray radiation during the treatment and safe guidance of the intervention tool a position identification element is disposed in the region of the catheter tip. In an exemplary embodiment, the position identification element is part of an electromagnetic location system.

In a related field, U.S. Patent Application Publication 2004/0049121 describes an apparatus for use in a brain of a subject, including an instrument, adapted to be inserted into the brain. A set of one or more electrodes, coupled to the instrument, are adapted to sense electrical activity of the brain and to transmit an electrical activity signal responsive thereto. A location sensor, adapted to be coupled to the instrument transmits a location signal indicative of a location of the instrument. A control unit, analyzes the electrical activity signal and the location signal. The control unit determines, responsive to the analysis, a position of the instrument with respect to an image of the brain, and electrophysiological information regarding tissue at the position.

### SUMMARY OF THE INVENTION

An embodiment of the present invention provides a location tracking system, including a location pad collar and a processor. The location pad collar is configured to be placed around a neck of a patient and includes one or more magnetic field generators configured to generate one or more magnetic fields within a head of the patient. The processor is configured to receive, in response to the one or more generated magnetic fields, one or more position signals that are indicative of a location of a distal end of a sheath of a probe inside a blood vessel in a brain of the patient. Based on the received position signals, the processor is configured to estimate the location of the distal end of the sheath in the brain relative to a clot in the brain.

In some embodiments, the processor is configured to receive the position signals from a magnetic position sensor fitted at the distal end of the sheath.

In some embodiments, the magnetic position sensor is formed on a flexible printed circuit board (PCB) wrapped around the distal end of the sheath.

In an embodiment, the processor is configured to estimate the location of the distal end of the sheath in the brain relative to the clot by registering the estimated location with one or more medical images that demonstrate the clot.

In another embodiment, the processor is additionally configured to correct the estimated location of the distal end of the sheath on the medical images so that the location of the distal end of the sheath conforms to imaged anatomy of the patient.

In some embodiments, the processor is configured to indicate to a user whether the distal end of the sheath has traversed the clot.

In some embodiments, the system further includes a reference position sensor configured to generate additional position signals that are indicative of head movement of the patient.

In an embodiment, the reference position sensor is attached to a forehead of the patient.

In an embodiment, the reference position sensor comprises a magnetic position sensor.

In another embodiment, the processor is configured to correct an estimation of the location of the distal end of the sheath based on the indicated head movement.

There is additionally provided, in accordance with an embodiment of the present invention a location tracking method, including placing around a neck of a patient a location pad collar, which includes one or more magnetic field generators configured to generate one or more magnetic fields within a head of the patient. One or more position signals that are indicative of a location of a distal end of a sheath of a probe inside a blood vessel in a brain of the patient are received in response to the one or more generated magnetic fields. The location of the distal end of the sheath in the brain relative to a clot in the brain is estimated based on the received position signals.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a cerebrovascular catheter-based position tracking system, in accordance with an embodiment of the present invention; and
Fig. 2 is a schematic cross-sectional view of a brain clot and a catheter, in accordance with an embodiment of the present invention; and
Fig. 3 is a flow chart that schematically illustrates a method for tracking a catheter in the brain, in accordance with an embodiment of the present invention.

### DETAILED DESCRIPTION OF EMBODIMENTS

### OVERVIEW

Embodiments of the present invention that are described hereinafter provide a system and method for tracking a distal end of a medical probe, e.g., a catheter sheath, as it is being advanced via the cerebrovascular system to engage a brain clot. The distal end of the probe typically comprises a distal end of a sheath and a distal end of a shaft, wherein in various embodiments different elements are be fitted at the distal end of the sheath and/or at the distal end of the shaft. The embodiments described herein refer mainly to a catheter sheath, by way of example.

In an example embodiment, a clot in the brain is removed by inserting and advancing a clot-removal device into a blood vessel of the brain, such as a clot removing stent. Typically, the clot is removed by first sliding the sheath of the catheter between the clot and the wall containing it (in other words, penetrating the clot), until the sheath traverses the clot. The stent is then passed through the sheath and then, upon exiting the sheath, expands to capture the clot. The sheath and the stent, along with the captured clot, are then withdrawn. In some embodiments, the sheath is fitted with an inflatable balloon proximally its distal end, wherein the balloon is inflated to anchor the sheath, so as to increase a penetrating force with which the distal end of the sheath can traverse or penetrate a clot.

In some embodiments, a location pad collar is placed around the neck of a patient. The location pad comprises magnetic field generators that are configured to generate magnetic fields within the head of the patient. A magnetic position sensor is fitted at a distal end of the sheath. In response to sensed magnetic fields, the position sensor generates position signals that are indicative of its location in the brain. A processor receives the position-indicative signals and uses them to estimate a location of the distal end of the sheath in the brain.

The location pad collar is configured to automatically compensate for a patient's head movement during location tracking, by the pad collar moving as a result of the head motion. In an embodiment, an additional reference position sensor may be attached to the forehead of the patient so as to generate additional position signals that are indicative of head movement. The processor may use the latter position signals to automatically compensate (i.e., correct) its estimation of the location of the distal end of the sheath based on the indicted head movement. In an embodiment, the reference sensor comprises a magnetic position sensor.

In an embodiment, the magnetically estimated location of the distal end of the sheath is registered with medical images of the brain, such as CT images that demonstrate the clot, allowing the distal end of the sheath to be presented on the images in relation with the imaged clot. The tracking allows, if necessary, correcting the position of the distal end of the sheath to conform to imaged anatomy, for example, to correctly show the distal end of the sheath as contained within a blood vessel.

In some embodiments, an indication presented on the images, of the location of the distal end of the sheath relative to the demonstrated brain-clot, is used for verifying (i.e., indicating) that the distal end of the sheath has traversed the clot. In some embodiment, upon receiving the indication that the distal end of the sheath has traversed the clot, a clot removal device fitted at the distal end of the shaft is deployed for removing the clot.

While X-ray fluoroscopy may also be used to track a probe as it is being advanced to the clot, fluoroscopy exposes the patient to relatively large doses of ionizing X-ray radiation, as well as large amounts of contrast agent. The physician is also repeatedly exposed to X-ray radiation, which, during numerous procedures, may accumulate to an undesired level.

The disclosed technique for tracking a probe while being advanced to a clot, and optionally during and after removing the clot, eliminates the need for potentially hazardous X-ray fluoroscopy. Furthermore, the disclosed technique may eliminate the need for a large and expensive catherization room to accommodate an X-ray based imaging modality.

### POSITION SENSOR ON BRAIN-CLOT REMOVAL SHEATH AND LOCATION PAD COLLAR SYSTEM DESCRIPTION

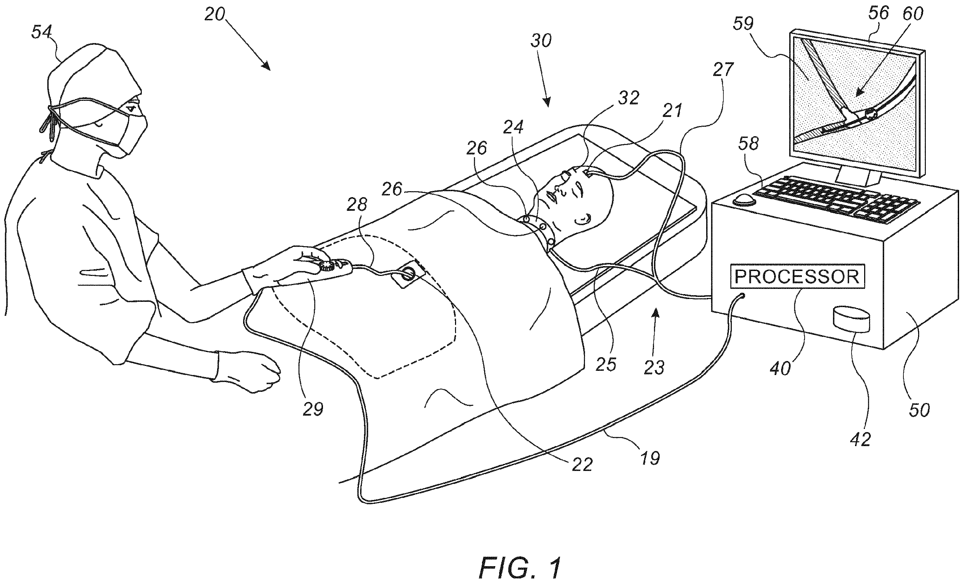

Fig. 1 is a schematic, pictorial illustration of a cerebrovascular catheter-based position tracking system 20, in accordance with an embodiment of the present invention. System 20 registers frames of reference of a CT image of a patient 32, herein assumed, by way of example, to comprise a fluoroscopic CT image, and of a magnetic tracking system 23 used to track a magnetic sensor within the patient's brain.

In system 20, a location pad 24, comprised of a magnetic tracking system, is implemented as a collar placed around the neck of patient 32, and which automatically compensates for patient head movement. Location pad 24 comprises magnetic field generators 26 which are fixed in positions relative to the head of patient 32 and which transmit alternating sinusoidal magnetic fields into a region 30 in which the head of patient 32 is located. A console 50 electrically drives generators 26 via a cable 25. In an embodiment, further compensation of head motion is provided by attaching a reference position sensor 21 to the patient's forehead. Console 50 is configured to receive signals from reference position sensor 21 via a cable 27.

By way of example, generators 26 of location pad 24 are arranged in an approximately circular shape around the neck of patient 22. However, any suitable alternate configuration for the generators of location pad 24 can be used.

A catheter controller handle 29, held by a physician 54 who is operating system 20, is connected to the proximal end of a catheter 28. The controller handle 29 allows the physician to advance and navigate catheter 28 in the brain, for example through an entry point 22 at a thigh artery of patient 32. Physician 54 navigates the distal end of catheter 28 using position signals from a magnetic position sensor fitted at a distal end of catheter 28, as further described below. Console 50 receives the position signals via a cable 19 that connects to catheter 28 via handle 29. The Carto® system produced by Biosense Webster, of Irvine, CA, uses a system similar to that described herein for finding the location and orientation of a magnetic position sensor in a brain region irradiated by magnetic fields.

Elements of system 20, including generators 26, are controlled by a system processor 40, comprising a processing unit communicating with one or more memories. Processor 40 may be mounted in console 50, which comprises operating controls 58 that typically include a keypad and/or a pointing device such as a mouse or trackball. Physician 54 uses operating controls on handle 29 to interact with the processor while performing the registration of system 20. During the registration process, an image 59 of a brain section is presented on a screen 56. Subsequent to the registration process, physician 54 uses the operating controls to advance the distal end of catheter 28 to a brain location 60 where a clot blocks an artery. The processor presents results of the catheter tracking procedure on screen 56.

Processor 40 uses software stored in a memory 42 to operate system 20. The software may be downloaded to processor 40 in electronic form, over a network, for example, or it may, alternatively or additionally, be provided and/or stored on non-transitory tangible media, such as magnetic, optical, or electronic memory.

Processor 40 uses the software, among other functions, to operate magnetic generators 26 of location pad 24. As stated above, the generators transmit sinusoidal alternating magnetic fields of different frequencies into region 30, including the head of patient 22, as well as inducing signals in sensor 32. The processor analyzes the signals to derive location and orientation values for the sensor, and thus for the distal end of catheter 28, measured with respect to a frame of reference defined by location pad 24.

Prior to the catheterization procedure, CT images of patient 22 are received at system 20, and the CT image data are stored in memory 42, for subsequent retrieval by processor 40 for the registration of the distal end of catheter 28. As is described below, the processor uses the stored data to present brain section image 59 on screen 56.

The system shown in Fig. 1 is chosen purely for the sake of conceptual clarity. Other system elements may be included, for example additional magnetic field generators arranged in a horseshoe shape around the head of patient 32.

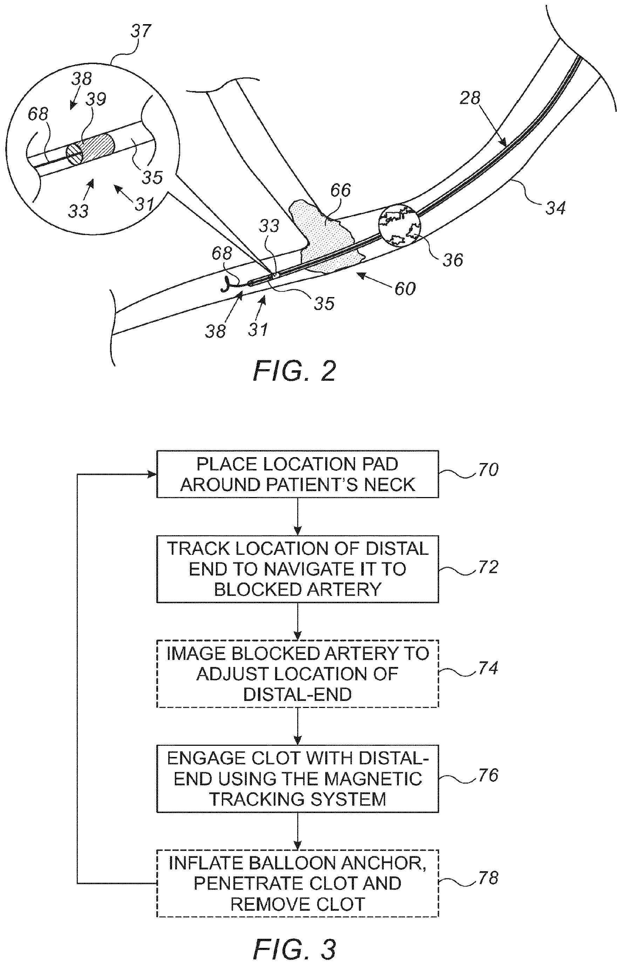

Fig. 2 is a schematic cross-sectional view of a brain clot 66 and catheter 28, in accordance with an embodiment of the present invention. As seen, clot 66 blocks blood flow from an artery 34. In some embodiments, catheter 28 is advanced through artery 34 to penetrate clot 66, in order to remove the clot. In an embodiment, a clot removal device 68 is fitted at a distal end 38 of the shaft of catheter 28, and is used for removing the clot after the device is navigated to a correct location inside artery 34. In an optional embodiment, catheter 28 is fitted with a balloon 36 that is configured to anchor a sheath 35 while catheter 28 moves through the clot, which, otherwise, may resist penetration and cause catheter 28 to retreat

As seen in Fig. 2, a distal end 31 of sheath 35 includes a magnetic position sensor 33, which is used for tracking distal end 31 in the brain, as described above. Magnetic position sensor 33 may contain a single-axis or triple-axis magnetic transducer.

Typically, distal end 31 would be advanced through a small diameter sheath 35 (e.g., in a range of five to ten French, 5-10Fr) to provide catheter 28 passage with sufficient maneuverability in narrow blood vessels. In some embodiments, sensor 33 may be formed on a flexible printed-circuit-board 39 wrapped around sheath 35 in order to conform to a small diameter of distal end 31 of sheath 35, as shown in an inset 37. Sensor 33 may be patterned and/or fitted with elements, e.g., a sensor coil made of patterned conductive lines, which is not shown for clarity of presentation.

The example illustration shown in Fig. 2 is chosen purely for the sake of conceptual clarity. Other architectures of distal end 31, and specifically of sensor 33, may be elaborated by a person skilled in the art, while performing the functions described herein.

Fig. 3 is a flow chart that schematically illustrates a method for tracking a catheter in the brain, in accordance with an embodiment of the present invention. The process may begin when physician 54 places location pad 24 around the neck of patient 32, and, optionally, places reference position sensor 21 (e.g., a magnetic position sensor) on the forehead of patient 32, at a preparation step 70. Next, physician 54 operates system 20 to track the location of distal end 31, while navigating sheath 35 through one or more blood vessels of the brain to bring distal end 31 to clot 66 that blocks artery 34, at a catheter navigation step 72.

In an optional embodiment, the physician may acquire new fluoroscopy images 59 while injecting a contrast agent, in order to register the tracked location of distal end 31 with a new image on display 59, for example while adjusting the location of distal end 31 of sheath 35 near clot 66, at a location adjusting step 74. Physician 54 operates system 20 to verify that distal end 31 is in proximity of clot 66, ready to engage the clot, at a tracking step 76. Next, in an optional embodiment, physician 54 inflates balloon anchor 36 and operates device 68 (fitted at distal end 38 of the shaft of catheter 28) to penetrate beyond clot 66, and remove clot 66 as the physician retracts distal end 38 through sheath 35 or retracts catheter 28 entirely (e.g. retracts together distal end 31 and distal end 38), at a clot removal step 78.

The example flow chart shown in Fig. 3 is chosen purely for the sake of conceptual clarity. In alternative embodiments physician 54 may perform additional or alternative diagnostic or therapeutic steps at the location of clot 66. For example, the physician may inject a medication at the location of clot 66 through catheter 28, may employ, for example, additional monitoring steps, either invasive or noninvasive, to verify the successful outcome of the procedure, and/or may apply other sensors fitted to distal end 31, for example, to acquire additional clinical data, such as local blood pressure.

Although the embodiments described herein mainly address cerebrovascular applications, the methods and systems described herein can also be used in other applications, such as in cardiovascular applications.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

### ASPECTS OF THE INVENTION

1. A location tracking method, comprising:
   placing around a neck of a patient a location pad collar, which comprises one or more magnetic field generators configured to generate one or more magnetic fields within a head of the patient;
   receiving, in response to the one or more generated magnetic fields, one or more position signals that are indicative of a location of a distal end of a sheath of a probe inside a blood vessel in a brain of the patient; and
   based on the received position signals, estimating the location of the distal end of the sheath in the brain relative to a clot in the brain.
2. The method according to aspect 1, wherein receiving the position signals comprises receiving the position signals from a magnetic position sensor fitted at the distal end of the sheath.
3. The method according to aspect 2, wherein the magnetic position sensor is formed on a flexible printed circuit board (PCB) wrapped around the distal end of the sheath.
4. The method according to aspect 1, wherein estimating the location of the distal end comprises estimating the location of the distal end of the sheath relative to the clot by registering the estimated location with one or more medical images that demonstrate the clot.
5. The method according to aspect 1, and comprising correcting the estimated location of the distal end of the sheath on the medical images so that the location of the distal end of the sheath conforms to imaged anatomy of the patient.
6. The method according to aspect 1, wherein estimating the location of the distal end of the sheath comprises indicating to a user whether the distal end of the sheath has traversed the clot.
7. The method according to aspect 1, and comprising receiving from a reference position sensor, additional position signals that are indicative of head movement of the patient.
8. The method according to aspect 7, wherein receiving the additional position signals comprises receiving the additional position signals from a reference position sensor that is attached to a forehead of the patient.
9. The method according to aspect 7, wherein receiving the additional position signals comprises receiving the additional position signals from a magnetic position sensor.
10. The method according to aspect 7, wherein estimating the location of the distal end comprises correcting an estimation of the location of the distal end of the sheath based on the indicated head movement.

## Claims

1. A location tracking system, comprising:
a location pad collar, which is configured to be placed around a neck of a patient and which comprises one or more magnetic field generators configured to generate one or more magnetic fields within a head of the patient; and
a processor configured to:
receive, in response to the one or more generated magnetic fields, one or more position signals that are indicative of a location of a distal end of a sheath of a probe inside a blood vessel in a brain of the patient; and
based on the received position signals, estimate the location of the distal end of the sheath in the brain relative to a clot in the brain.

2. The system according to claim 1, wherein the processor is configured to receive the position signals from a magnetic position sensor fitted at the distal end of the sheath.

3. The system according to claim 2, wherein the magnetic position sensor is formed on a flexible printed circuit board (PCB) wrapped around the distal end of the sheath.

4. The system according to claim 1, wherein the processor is configured to estimate the location of the distal end of the sheath in the brain relative to the clot by registering the estimated location with one or more medical images that demonstrate the clot.

5. The system according to claim 4, wherein the processor is additionally configured to correct the estimated location of the distal end of the sheath on the medical images so that the location of the distal end of the sheath conforms to imaged anatomy of the patient.

6. The system according to claim 1, wherein the processor is configured to indicate to a user whether the distal end of the sheath has traversed the clot.

7. The system according to claim 1, and comprising a reference position sensor configured to generate additional position signals that are indicative of head movement of the patient.

8. The system according to claim 7, wherein the reference position sensor is attached to a forehead of the patient.

9. The system according to claim 7, wherein the reference position sensor comprises a magnetic position sensor.

10. The system according to claim 7, wherein the processor is configured to correct an estimation of the location of the distal end of the sheath based on the indicated head movement.
